# EUROPÄISCHE PATENTSCHRIFT

(11) **EP 0 934 080 B1**
(45) Veröffentlichungstag und Bekanntmachung des Hinweises auf die Patenterteilung: **12.06.2002**
(21) Anmeldenummer: 97911187.9
(22) Anmeldetag: 07.10.1997
(51) Int. Cl.: A61K 47/22, A61K 7/00, C07D 207/263, C07D 211/76, C07D 223/10, C07D 227/087, C07D 265/32, C07D 263/18, C07D 401/06, C07D 403/06, C07D 413/06

(54) **1,3-BIS-(N-LACTAMYL)PROPANE UND DEREN PHARMAZEUTISCHE UND KOSMETISCHE VERWENDUNG**
1,3-BIS-(N-LACTAMYL) PROPANE AND THE PHARMACEUTICAL AND COSMETIC USE THEREOF
1,3-BIS-(N-LACTAMYL)PROPANE ET SON UTILISATION EN PHARMACIE ET EN COSMETIQUE

(30) Priorität: 08.10.1996 DE 19641437
(43) Veröffentlichungstag der Anmeldung: 11.08.1999
(73) Patentinhaber: BASF AKTIENGESELLSCHAFT, 67056 Ludwigshafen (DE)
(72) Erfinder: BREITENBACH, Jörg, D-68199 Mannheim (DE); LANG, Siegfried, D-67071 Ludwigshafen (DE); KRATZ, Detlef, D-69121 Heidelberg (DE)
(74) Vertreter: Kinzebach, Werner, Dr.
(86) Internationale Anmeldenummer: EP9705499
(87) Internationale Veröffentlichungsnummer: WO9815291

(56) Entgegenhaltungen:
- DE-A- 2 239 920
- DE-B- 1 053 736
- US-A- 3 989 815
- US-A- 5 326 880

## Beschreibung

Die vorliegende Erfindung betrifft 1,3-Bis-(N-lactamyl)propane und deren Verwendung als Lösungsmittel in pharmazeutischen und kosmetischen Mitteln.

Bei der Entwicklung einer optimalen Darreichungsform für ein Arzneimittel steht die Forderung nach einer guten biologischen Verfügbarkeit im Vordergrund. In der Regel bieten dafür Lösungen des verwendeten Wirkstoffes die günstigsten Voraussetzungen, unabhängig davon, ob es sich um ein Injektionspräparat, eine flüssige orale Arzneiform oder beispielsweise eine Weichgelatine-Kapselzubereitung handelt.

Viele Arzneimittel sind allerdings zuwenig wasserlöslich, um beispielsweise parenteral in Form einer wäßrigen Lösung verabreicht werden zu können. Es wird daher häufig auf andere, beispielsweise organische, wäßrig-organische oder ölige Lösungsmittel zurückgegriffen.

Bei der Auswahl geeigneter Lösungsmittel ist es wichtig, auf die Erfüllung bestimmter Erfordernisse zu achten. Das verwendete Solvens darf weder toxisch sein, noch darf es die Toxizität des in ihm gelösten Wirkstoffes erhöhen. Selbstverständlich muß das Solvens mit dem Wirkstoff kompatibel sein und darf nicht zu Inaktivierung oder Zersetzung führen. Weiterhin darf das Solvens auch nicht zu einer Reizung, Sensibilisierung oder einer allergischen Reaktion Anlaß geben und es sollte auch für die Hitzesterilisation geeignet sein. Für parenterale Zwecke sollte die Viskosität niedrig genug sein, um eine Injektion mit einer relativ dünnen Nadel durchführen zu können.

Außerdem ist die Mischbarkeit mit anderen Lösungsmitteln, wie physiologischer Kochsalzlösung oder einer Infusionslösung für die Zubereitung eines Injektionspräparates oder dem Trägeröl einer Weichgelatine-Kapselzubereitung von Bedeutung.

Bekannte Lösungsmittel neben Wasser sind beispielsweise niedermolekulare Polyethylenglykole, 1,2-Propylenglykol, Ethanol, Glycerin, Benzylalkohol, Dimethylacetamid oder Neutralöle, wie Paraffin, Rizinusöl oder Triglycerid-Gemische von gesättigten Pflanzenfettsäuren.

Die Eigenschaften der oben genannten Lösungsmittel befriedigen jedoch nicht immer. So kann die Mischbarkeit mit anderen Lösungsmitteln eingeschränkt sein oder das zum Lösen des Wirkstoffes benötigte Volumen an Lösungsmitteln ist so groß, daß die physiologische Verträglichkeit nicht mehr gegeben ist. Das vielfach verwendete Propylenglykol ist beispielsweise nicht mit fetten Ölen mischbar. Benzylalkohol hat den Nachteil, daß er nur zu 4 % in Wasser löslich ist. Glycerin ist zwar mischbar mit Wasser, aber es fehlt die Löslichkeit in fetten Ölen. Außerdem ist die für Glycerin maximal anwendbare Konzentration aus Verträglichkeitsgründen auf etwa 5 % beschränkt. Dimethylacetamid besitzt sehr gute Lösungseigenschaften, aber die Verträglichkeit ist verhältnismäßig schlecht, so daß eine Anwendung bei Menschen nicht unbedenklich erscheint.

Die Verwendung von Pyrrolidonderivaten, wie 2-Pyrrolidon, N-Methylpyrrolidon und Hydroxyethylpyrrolidon als Lösungsmittel oder Co-Lösungsmittel ist bekannt.

Die EP-A-271 374 beschreibt beispielsweise wäßrige, antibiotische Zusammensetzungen zum veterinären Gebrauch, bei denen ein Tetracyclin in N-(Hydroxyethyl)-2-pyrrolidon gelöst wird.

Die EP-A-626 171 offenbart Injektionslösungen für die intravenöse oder intramuskuläre Verabreichung von Oxytetracyclin oder Doxycyclin, wobei die Lösungsmittelphase aus N-Methylpyrrolidon und/oder 2-Pyrrolidon gebildet wird.

Ein Gemisch aus 2-Pyrrolidon und N-Methyl-2-pyrrolidon kennzeichnet den pharmazeutischen Träger eines Mittels zur kosmetischen und therapeutischen Behandlung von menschlichen und tierischen Lebewesen, der in der DE 26 15 140 beschrieben ist.

Obwohl die oben beschriebenen, auf Pyrrolidonderivaten basierenden Formulierungen gute Löslichkeitseigenschaften aufweisen, erfüllen sie die an Arzneimittel gestellten hohen Anforderungen nicht, da die physiologische Verträglichkeit nicht in zufriedenstellendem Maße gegeben ist. Darüberhinaus sind die Resorptionszeiten relativ lang und die damit erzielten Serumspiegel verbesserungswürdig.

Auch die im Stand der Technik beschriebenen Bis- bzw. Poly-(N-pyrrolidonyl)alkane genügen nicht in ausreichendem Maß den an pharmazeutische Lösungsmittel gestellten, hohen Anforderungen. So werden die asymetrischen Polypyrrolidonyl-Verbindungen der US 3,989,815 überhaupt nicht für die Verwendung als Lösungsmittel vorgesehen, sondern finden nur als Dispergiermittel Anwendung. Die als Träger topisch verabreichter pharmazeutischer Mittel in der US 5,326,880 beschriebenen Bis-(N-pyrrolidonyl)alkane lösen viele Wirkstoffe nur in begrenztem Umfang. Sie sind somit für pharmazeutische Zubereitungen, bei denen es auf eine gute Lösbarkeit des Wirkstoffes ankommt, nicht geeignet.

Der vorliegenden Erfindung liegt daher die Aufgabe zugrunde, Lösungsmittel für pharmazeutische Zubereitungen bereitzustellen, die neben einem hervorragenden Lösevermögen für den verwendeten Wirkstoff auch eine ausgezeichnete Mischbarkeit mit anderen Lösungsmitteln und eine gute physiologische Verträglichkeit bieten.

Es wurde nun überraschenderweise gefunden, daß bestimmte, vorzugsweise asymmetrische 1,3-Bis-(N-lactamyl)propane eine Vielzahl in Wasser schwer löslicher Wirkstoffe in hervorragender Weise lösen und darüberhinaus universell verwendbar sind, da sie sich in jedem Verhältnis mit anderen, üblichen pharmazeutischen Lösungsmitteln mischen. Die erfindungsgemäßen Verbindungen sind chemisch indifferent und weisen eine hohe physiologische Verträglichkeit auf.

Gegenstand der vorliegenden Erfindung ist die Verwendung wenigs tens eines 1,3-Bis-(N-lactamyl)propans der allgemeinen Formel I 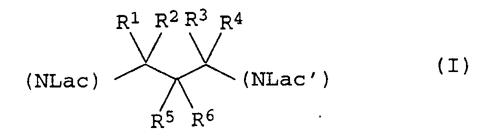 worin die Reste R¹, R², R³, R⁴, R⁵ und R⁶ unabhängig voneinander für Wasserstoff, Alkyl, Cycloalkyl, Aryl oder Aralkyl stehen und wenigstens einer dieser Reste nicht für Wasserstoff steht, (NLac) und (NLac') gleich oder verschieden sind und für N-Lactamylreste stehen, als Lösungsmittel in pharmazeutischen oder kosmetischen Mitteln.

Der Ausdruck "Alkyl" umfaßt geradkettige oder verzweigte Alkylgruppen, vorzugsweise C₁-C₁₀-Alkyl, insbesondere C₁-C₆-Alkyl und besonders bevorzugt C₁-C₃-Alkyl, wie Methyl, Ethyl, n- und i-Propyl, n-, i- oder t-Butyl, n-Pentyl, Neopentyl oder n-Hexyl, etc.

"Cycloalkyl" umfaßt cyclische Alkylgruppen, wie Cyclopropyl, Cyclobutyl, Cyclopentyl, Cyclohexyl, Cycloheptyl, Cyclooctyl sowie polycyclische Reste wie Norbornyl, Camphyl, Pinanyl oder Decahydronaphthyl, wobei sowohl die mono- als auch polycyclischen Reste durch einen oder mehrere C₁-C₄-Alkylgruppen substituiert sein können.

Der Ausdruck "Aryl" umfaßt aromatische Reste, vorzugsweise Naphthyl und insbesondere Phenyl.

Unter "Aralkyl" versteht man Arylgruppen, die über eine C₁-C₄-Alkyleneinheit mit dem Grundgerüst verbunden sind. "Aralkyl" steht vorzugsweise für Benzyl.

Unter "Alkylen" versteht man lineare oder verzweigte, zweiwertige Alkylreste, vorzugsweise C₁-C₆-Alkylen und insbesondere C₁-C₄-Alkylen, wie Methylen, 1,1- und 1,2-Ethylen, 1,1-, 1,2-, 1,3- und 2,2-Propylen, 2-Methyl-1,1-propylen etc.

Der Ausdruck "N-Lactamyl" steht für N-substituierte mono- oder polycyclische Lactame, die mit C₁-C₄-Alkylgruppen, C₁-C₄-Alkoxygruppen oder Halogen substituiert und/oder mit Aromaten kondensiert sein und/oder weitere Heteroatome, vorzugsweise Stickstoff oder Sauerstoff, im Lactam-Ring enthalten können. Besonders bevorzugte N-Lactamylreste leiten sich von Pyrrolidon, Piperidon, δ-oder ε-Caprolactam, Oxazolidin-4-on oder Tetrahydro-1,4-oxazin-3-on und insbesondere von Pyrrolidon ab.

In bevorzugt als Lösungsmittel in pharmazeutischen Mitteln zur Anwendung kommenden erfindungsgemäßen 1,3-Bis-(N-lactamyl)-propanen steht wenigstens einer der Reste R¹, R², R³, R⁴, R⁵ und R⁶ für Alkyl, während die übrigen dieser Reste für Wasserstoff stehen.

Insbesondere geeignet sind Verbindungen der Formel I, worin nur einer der Reste R¹, R², R³, R⁴, R⁵ und R⁶ nicht für Wasserstoff steht, wobei R¹ in diesem Fall bevorzugt ist und insbesondere für Alkyl steht.

In einer besonderen Ausführungsform verwendet man 1,3-Bis-(N-lactamyl)butane, d.h. Verbindungen der Formel I, worin R¹ für Methyl steht.

Die Lactamylgruppen (NLac) bzw. (NLac') in Formel I können gleich oder verschieden sein, bevorzugt sind sie jedoch gleich.

Eine besondere Verbindungsklasse zur Verwendung als Lösungsmittel in pharmazeutischen Zubereitungen stellen diejenigen Verbindungen der oben beschriebenen Art dar, die kein Symmetriezentrum aufweisen. Solche Verbindungen ergeben sich dadurch, daß einerseits die substituierte Propyleneinheit per se asymmetrisch ist und/oder die Lactamylreste NLac und NLac' verschieden sind. Bevorzugt sind Verbindungen, in denen die Propyleneinheit asymmetrisch ist und die beiden Lactamylreste die gleiche Bedeutung besitzen.

Ganz besonders bevorzugt sind folgende Verbindungen:
1,3-Bis(pyrrolidon-l-yl)butan
1,3-Bis(piperidon-l-yl)butan
1,3-Bis(caprolactam-l-yl)butan
1,3-Bis(tetrahydro-1,4-oxazin-3-on-1-yl)butan
1,3-Bis(oxazolidin-4-on-3-yl)butan
1-(Caprolactam-1-yl)-3-(piperidon-1-yl)butan
3-(Caprolactam-l-yl)-1-(piperidon-l-yl)butan
1-(Caprolactam-l-yl)-3-(pyrrolidon-1-yl)butan
3-(Caprolactam-1-yl)-1-(pyrrolidon-l-yl)butan
1-(Caprolactam-l-yl)-3-(oxazolidin-4-on-3-yl)butan
3-(Caprolactam-1-yl)-1-(oxazolidin-4-on-3-yl)butan
1-(Caprolactam-1-yl)-3-(tetrahydro-1,4-oxazin-3-on-4-yl)butan
3-(Caprolactam-l-yl)-1-(tetrahydro-1,4-oxazin-3-on-4-yl)butan
1-(Oxazolidin-4-on-3-yl)-3-(piperidon-1-yl)butan
3-(Oxazolidin-4-on-3-yl)-1-(piperidon-1-yl)butan
1-(Oxazolidin-4-on-3-yl)-3-(pyrrolidon-1-yl)butan
3-(Oxazolidin-4-on-3-yl)-1-(pyrrolidon-1-yl)butan
1-(Oxazolidin-4-on-3-yl)-3-(tetrahydro-1,4-oxazin-3-on-4-yl)butan
3-(Oxazolidin-4-on-3-yl)-1-(tetrahydro-1,4-oxazin-3-on-4-yl)butan
1-(Piperidon-1-yl)-3-(pyrrolidon-1-yl)butan
3-(Piperidon-l-yl)-1-(pyrrolidon-1-yl)butan
1-(Piperidon-1-yl)-3-(tetrahydro-1,4-oxazin-3-on-4-yl)butan
3-(Piperidon-1-yl)-1-(tetrahydro-1,4-oxazin-3-on-4-yl)butan
1-(Pyrrolidon-1-yl)-3-(tetrahydro-1,4-oxazin-3-on-4-yl)butan
3-(Pyrrolidon-l-yl)-1-(tetrahydro-1,4-oxazin-3-on-4-yl)butan.

Einige der oben beschriebenen, erfindungsgemäß geeigneten Verbindungen weisen ein oder mehrere asymmetrische Kohlenstoffatome auf und können daher in verschiedenen stereochemischen Formen existieren. Sowohl die einzelnen Isomere (Enantiomere oder Diastereomere), als auch deren Mischungen sind Gegenstand dieser Erfindung.

Die Herstellung der Verbindungen mit der allgemeinen Formel I ist im Prinzip bekannt. So beschreibt Breitenbach et al. (Naturwissenschaften 42, 1955, 155; 440) die Dimerisierung von N-Vinylpyrrolidon unter sauren Reaktionsbedingungen und die nachfolgende Hydrierung des erhaltenen 1,3-Bis-(N-pyrrolidonyl)-1-butens zum 1,3-Bis-(N-pyrrolidonyl)butan. In Anlehnung an diese Vorgehensweise lassen sich gemäß Schema I durch sauer katalysierte Reaktion der Vinyllactame der allgemeinen Formel IIa bzw. IIb, worin Rₐ, R_{b}, R_{c} und R_{d} unabhängig voneinander für Wasserstoff, Alkyl, Cycloalkyl, Aryl oder Aralkyl stehen und (NLac) sowie (NLac') für die oben erwähnten Lactamylreste stehen, die Propene der allgemeinen Formel IIIa-d herstellen. Die Verbindungen III a-d werden anschließend durch Hydrierung in die entsprechenden Verbindungen der allgemeinen Formeln I a-d überführt. Die Verbindungen I a-d entsprechen jenen Verbindungen der Formel I, in denen zwei der an benachbarten Kohlenstoffen befindlichen Reste, beispielsweise R¹ und R⁵, für Wasserstoff stehen. Bei Verwendungen von Mischungen zweier Vinyllactame der allgemeinen Formel II sind sowohl die gemischten Reaktionsprodukte sowie die beiden Produkte einer Reaktion der Vinyllactame II mit sich selber zugänglich. Die Verbindungen können sowohl auf der Stufe der Propene IIIa-d als auch auf der Stufe der Endprodukte Ia-d getrennt werden, was jedoch für die Herstellung der erfindungsgemäßen Lösungsmittel nicht immer erforderlich ist. Bevorzugt werden auf diesem Wege die Verbindungen I hergestellt, die durch Umsetzung der Vinyllactame IIa bzw. IIb mit sich selbst erhalten werden (Ia bzw. Id).

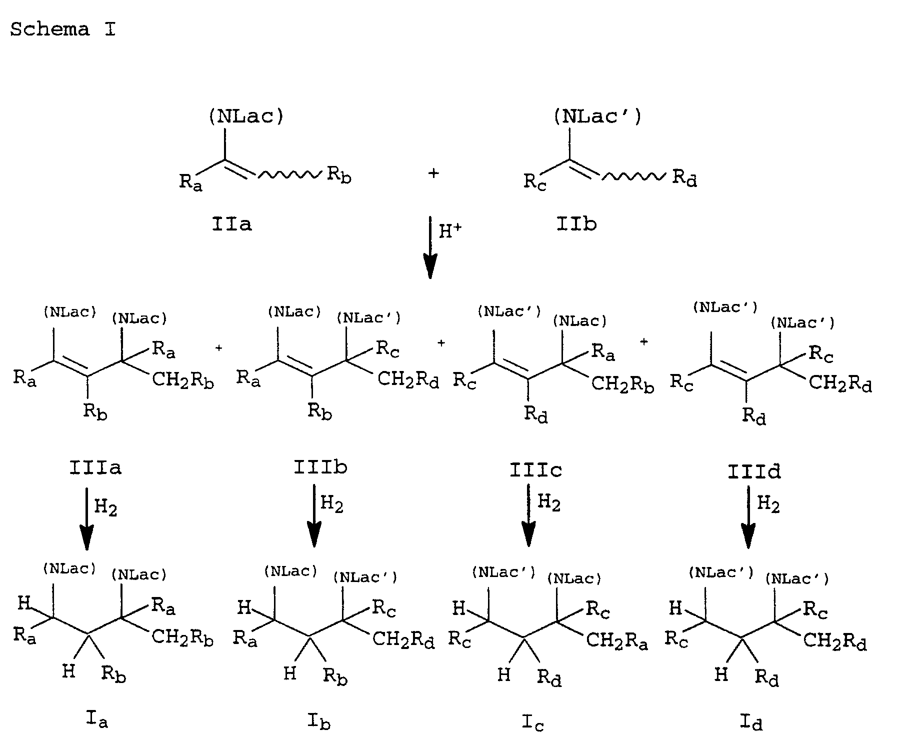

Die eingesetzen Vinyllactame der allgemeinen Formel IIa bzw. IIb sind bekannt oder können nach allgemeinen Verfahren in Anlehnung an die Herstellung der bekannten Vinyllactame II hergestellt werden. Hinsichtlich der Konfiguration der olefinischen Doppelbindungen in der Verbindung der allgemeinen Formel II bestehen keine Einschränkungen.

Die Umsetzung der Vinyllactame II zu den Bis-(N-lactamyl)-propenen III kann nach den üblichen Methoden der sauer katalysierten Dimerisierung von Olefinen mit einer Protonensäure, einem Lewis-Säure-Katalysator oder Ziegler-Katalysatoren erfolgen (siehe z.B. J. March, Advanced Organic Chemistry 3^{rd} ed., S. 709 und dort zitierte Literatur) erfolgen. Die Umsetzung kann sowohl in Substanz als auch in einem inerten Lösungsmittel, z.B. einem halogenierten Kohlenwasserstoff, wie CH₂Cl₂, Aromaten, wie Toluol, Ether, wie Diethylether, Dioxan oder Tetrahydrofuran oder einem sonstigen Lösungsmittel, das für elektrophile Reaktionen geeignet ist, durchgeführt werden. Bevorzugte Katalysatoren sind starke Protonensäuren, vorzugsweise solche mit wenig nucleophilen, korrespondierenden Anionen, wie Schwefelsäure, Sulfonsäuren, z.B. p-Toluolsulfon-säure, Methansulfonsäure oder Trifluormethansulfonsäure, Perchlorsäure oder Chlorwasserstoff. Die Säure wird vorzugsweise in substöchiometrischer Menge, bezogen auf die Vinyllactame, insbesondere in katalytischer Menge und besonders bevorzugt in Mengen bis 5 Mol % eingesetzt. Die Umsetzung wird vorzugsweise bei Temperaturen im Bereich von -20° bis +60° C und insbesondere im Bereich von 10° bis 40° C durchgeführt.

Der anschließende Hydrierungsschritt kann nach den üblichen Verfahren der Hydrierung olefinischer Doppelbindungen (siehe J. March, Advanced Organic Chemistry, 3rd ed., S. 691-708 und dort zitierte Literatur), vorzugsweise durch katalytische Hydrierung mit Wasserstoff an Übergangsmetall-Katalysatoren erfolgen. Aufgrund ihres Enamincharakters weisen die Doppelbindungen im Propenteil von III eine hohe Reaktivität auf, die es erlaubt, diese Doppelbindung selektiv in Gegenwart anderer, weniger reaktiver Doppelbindungen zu hydrieren.

Ein anderer Weg zu den 1,3-Bis-(N-lactamyl)propanen besteht in der Umsetzung von 1,3-Diaminen der allgemeinen Formel IV, worin R¹, R², R³, R⁴, R⁵ und R⁶ die oben für I angegebenen Bedeutungen besitzen, mit den geeigneten Lactonen (siehe Schema II; OLac steht für das dem jeweiligen N-Lactamylrest (N-Lac) entsprechende Lacton).

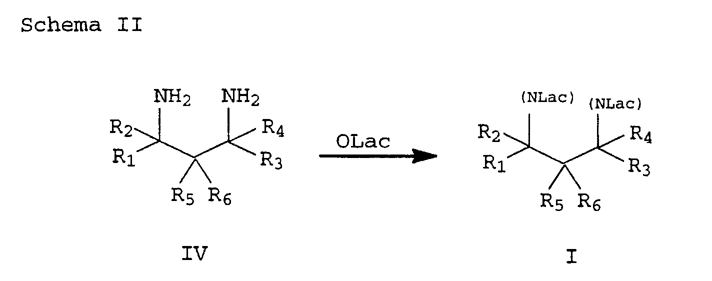

In der US-A 5,326,880 ist diese Vorgehensweise am Beispiel der Herstellung des 1,3-Bis-(N-pyrrolidonyl)pentan beschrieben. Die dort angegebene Vorgehensweise kann auf die Herstellung der erfindungsgemäßen Verbindungen der allgemeinen Formel I angewendet werden.

Eine weitere Möglichkeit zur Herstellung der Verbindungen der allgemeinen Formel I geht von 1,3-funktionalisierten Verbindungen der allgemeinen Formel V aus, worin X bzw. Y, die gleich oder verschieden sein können, für geeignete Abgangsgruppen, z.B. Halogenid, Tosylat, Brosylat oder Trifluormethansulfonat, stehen und R¹, R², R³, R⁴, R⁵ und R⁶ die oben genannten Bedeutungen besitzen. Derartige Verbindungen werden mit geeigneten N-Lactamylanionen zu den 1,3-Bis-(N-lactamyl)propanen der allgemeinen Formel I umgesetzt (Schema III). Die jeweiligen Lactamylanionen (NLac)- sind in der Regel durch Behandlung der Lactame (N-NLac) mit geeigneten Basen, wie Alkali- oder Erdalkalihydroxiden, Alkoholaten, Alkalihydriden oder -amiden zugänglich. Die Umsetzung der Verbindungen V mit den Anionen der Lactame kann nach den üblichen Methoden der N-Alkylierung von Amiden (vgl. J. March, Advanced Organic Chemistry, 3^{rd} ed., S. 377 ff. und dort zitierte Literatur) durchgeführt werden. Dabei können die 1,3-di-funktionalisierten Verbindungen V sowohl mit den Lactamen in Gegenwart einer Base oder aber mit den zuvor generierten Anionen der Lactame umgesetzt werden. Sind X und Y in ihrer Reaktivität verschieden, besteht insbesondere in letzterem Fall die Möglichkeit, Verbindungen der allgemeinen Formel I mit verschiedenen Lactamylresten zu erhalten.

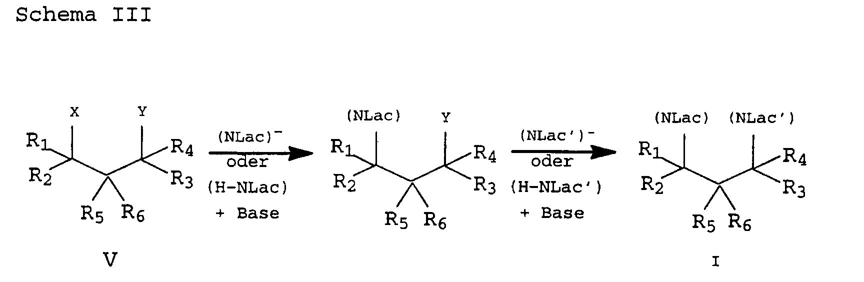

Geeignete 1,3-difunktionalisierte Verbindungen sind in der organischen Synthese häufig eingesetzte Ausgangsmaterialien und beispielsweise ausgehend von Aldolen durch Umwandlung der funktionellen Gruppen nach bekannten Verfahren oder ausgehend von Cyclopropanen durch halogenierende Spaltung der Cyclopropaneinheit zugänglich.

Gegenstand der vorliegenden Erfindung sind auch 1,3-Bis(N-Lactamyl)propane der allgemeinen Formel Ia 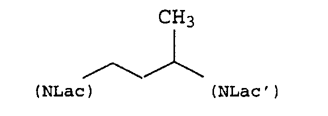 worin (NLac) und (NLac') gleich oder verschieden sind und für N-Lactamylreste stehen.

Bevorzugt leiten sich die N-Lactamylreste der vorstehend genannten Verbindungen von inbesondere unsubstituierten Pyrrolidonen, Piperidonen, δ- oder ε-Caprolactam, Tetrahydro-1,4-oxazin-3-onen oder Oxazolidin-4-onen ab. Besonders bevorzugt sind Pyrrolidon, Piperidon oder ε-Caprolactam. Vorzugsweise besitzen (NLac) und NLac') die gleiche Bedeutung.

Bevorzugt stehen die N-Lactamylreste für Pyrrolidon. Interessante Verbingungen ergeben sich aber auch, falls wenigstens ein und insbesondere beide N-Lactamylreste, die gleich oder verschieden sein können, einen wenigstens 6-gliedrigen Ring aufweisen.

Die erfindungsgemäße Verwendung der 1,3-Bis-(N-lactamyl)propane der allgemeinen Formel I als Lösungsmittel erstreckt sich auf alle, dem Fachmann bekannten pharmazeutischen Zubereitungen, bei denen der (oder die) Wirkstoff(e) in gelöster Form zur Anwendung kommen. Solche Formulierungen können oral, ophthalmologisch, rektal, parenteral (beispielsweise intravenös, intramuskulär, subcutan, intradermal, intraperitoneal, intrathorakal, intraarteriell, intracardial, intraspinal, intrasynovial usw), intraarticulär, topisch, nasal oder buccal verabreicht werden. Geeignete Zubereitungsformen umfassen Kapseln, beispielsweise Weichgelatine-Kapseln, Einreibemittel, Lotionen, Applikationen, Cremes, Salben, Gele, Tropfen, selbstaustreibende Formulierungen und Sprayformulierungen.

Insbesondere werden die erfindungsgemäßen Verbindungen bei der Zubereitung von pharmazeutischen Mitteln zur systemischen und insbesondere parenteralen sowie zur topischen Anwendung eingesetzt. Besonders gut eignen sich die Verbindungen der Formel I als Lösungsmittel von Injektabilia, beispielsweise Injektionsund Infusionszubereitungen und im Füllgut für Kapseln, beispielsweise Weichgelatine-Kapseln. Besonders vorteilhaft haben sich parenterale Zubereitungen erwiesen, welche die erfindungsgemäßen Verbindungen in Kombination mit Polyvinylpyrrolidon erhalten. Der Anteil an Polyvinylpyrrolidon liegt vorzugsweise im Bereich von 1 bis 70 Gew.-%, vorzugsweise 5 bis 60 Gew.-%, bezogen auf das Gesamtgewicht der Kombination. Bei Verwendung einer derartigen Kombination lassen sich parenterale Zubereitungen, insbesondere Injektabilia, mit langanhaltender Wirkung herstellen.

In topischen Präparaten können die erfindungsgemäßen Verbindungen die Resorption von Wirkstoffen beschleunigen.

Gegenstand der vorliegenden Erfindung sind auch pharmazeutische Mittel, die wenigstens ein 1,3-Bis-(N-lactamyl)propan der allgemeinen Formel I enthalten. Neben diesen erfindungsgemäßen Verbindungen enthält das pharmazeutische Mittel wenigstens einen Wirkstoff und ggf. weitere, übliche Träger- und/oder Hilfsstoffe.

Die Wirkstoffmenge pro Dosiseinheit und die Konzentration kann in weiten Grenzen variieren. Die einzige Bedingung ist, daß sie zur Erzielung der gewünschten Wirkung ausreichen. Auch Wirkstoff-Kombinationen können eingesetzt werden. Wirkstoffe im Sinne der Erfindung sind auch Vitamine und Mineralstoffe, sowie Pflanzenbehandlungsmittel und Insektizide. Zu Wirkstoffen im Sinne der Erfindung gehören auch Peptidtherapeutika. Besonders geeignet sind hydrophile Wirkstoffe.

Mit den erfindungsgemäßen Lösungsmitteln lassen sich beispielsweise folgende Wirkstoffe lösen:

Acebutolol, Acetylcystein, Acetylsalicylsäure, Acyclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlorpheniramin, Chlortalidon, Cholin, Cyclosporin, Cilastatin, Cimetidin, Ciprofloxacin, Cisapride, Cisplatin, Clarithromycin, Clavulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxocyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Iohexol, Iopamidol, Isosorbid-Dinitrat, Isosorbid-Mononitrit, Isotretinoin, Ketotifen, Ketoconazol, Ketoprofen, Ketorolac, Labatalon, Lactulose, Lecithin, Levocarnitin, Levodopa, Levoglutamid, Levonorgestrel, Levothyroxin, Lidocain, Lipase, Lipramin, Lisinopril, Loperamid, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Phenoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Promocriptin, Propafenon, Propranolol, Proxyphyllin, Pseudoephedrin, Pyridoxin, Quinidin, Ramipril, Ranitidin, Reserpin, Retinol, Riboflavin, Rifampicin, Rutosid, Saccharin, Salbutamol, Salcatonin, Salicylsäure, Simvastatin, Somatropin, Sotalol, Spironolacton, Sucralfat, Sulbactam, Sulfamethoxyzol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Volinsäure, Zidovudin.

Bevorzugt werden Wirkstoffe, die sich in den erfindungsgemäßen Lösungsmitteln in besonders hoher Konzentration lösen lassen. Hierzu zählen Acetylcystein, Acetylsalicylsäure, Anipamil x HCl, Butazolidin, Captopril, Esupron, Flecainid, Nexopamil, Nitrofurantoin, (+)-Pseudoephedrin x HCl, Selegilin x HCl, Hydantoine, wie 5,5-Diphenylhydantoin, Benzodiazepine, wie Diazepam, Chlordiazepoxid, Pyrazolonderivate, wie Diphenylbutazon, Aminophenazen, Dimethylaminophenazen, Carbamate, wie Meprobamat, Barbiturate, wie Phenobarbital, Pentobarbital, Aminobarbital, Antibiotika, wie Amoxicillin, Ciprofloxazin, Chloramphenicol, Oxytetracycline, Virustatika wie Acyclovir, Famcyclovir, sowie Antimycotika, wie Ketoconazol, Fluconazol und Itraconazol, Sulfonamide, wie Sulfamethoxazol, Sulfamoxol, Sulfathiazin, Steroide, wie Prednisolon, Cyproteronacetat, Hydroprogesteroncapronat, Sulfonamidpotentiatoren, wie Trimethoprim, nichtsteroidale Antirheumatika (NSAR), wie Ibuprofen, Diclofenac, Mefenaminsäure oder Theophyllin. Die fettlöslichen Vitamine A, E, K und ihre Derivate, wie Vitamin-A-palmitat, -acetat oder -propionat, Vitamin-E-acetat oder -nikonitat lassen sich sogar in jedem Verhältnis mit den erfindungsgemäßen Verbindungen mischen; gleiches gilt auch für Vitamin C. Von Vorteil ist auch die niedrige Viskosität, die sich bei Verwendung von Ölen oder halbsynthetischen Fettsäureestern häufig unvorteilhaft auswirkt. Weiterhin geeignet sind die Klasse des β-Blocker, Ca-Antagonisten, ACE-Hemmer, α-Blocker, H2-Antagonisten, Lipidsenker, Antihistaminika, ZNS-Antiepileptika und ZNS-Psychopharmaka.

Insbesondere geeignet sind Benzocain, Chloramphenicol, Cyclosporin, Clotrimazol, 5,5-Diphenylhydantoin, Fenofibrat, Furosemid, Gallopamil x HCl, Glibenclamid, Hydrochlorothiazid, Ketoprofen, Ibuprofen, Indometacin, Naphtidrofuryl, Nifedipin, Nitrendipin, Omeprazol, Paracetamol, Pentoxifyllin, Prazosin, Propafenon x HCl, Sulfamethazine, Sulfamethoxazol, Sulfathiazol, Sulindac, Tolbutamid, Tramadol und Zotepin.

Die Auswahl geeigneter Träger- und Hilfsstoffe richtet sich der nach Art des Wirkstoffes und insbesondere der Zubereitungsform.
Übliche, im Rahmen der vorliegenden Erfindung geeignete Trägeroder Hilfsstoffe sind beispielsweise Lösungsmittel, die mit den erfindungsgemäßen Lösungsmitteln mischbar sind, wie Wasser, Ethanol, Glycerin, 1,2-Propylenglykol, niedermolekulare Polyethylenglykole, Tetraglykol, Dimethylacetamid, Dimethylformamid, Benzylalkohol, Neutralöle, wie Paraffin, Olivenöl, Sesamöl, Arachisöl, Rizinusöl oder Triclycerid-Gemische von gesättigten Pflanzenfettsäuren;
Salze zur Tonizitätseinstellung, um bevorzugt isotonische Lösungen zu erhalten;
Puffer zur Einstellung des pH, um insbesondere Formulierungen mit physiologischen pH zu erhalten;
Mittel zur Erhöhung der Viskosität, z.B. bei topischer Anwendung, wie Polyethylenglykole, Polyvinylpyrrolidon oder Polyacrylate und Celluloseether;
Stabilisierungsmittel, wie Antioxidantien zum Schutz oxidationsempfindlicher Arzneistoffe, beispielsweise Sulfite, sulfoxylsaures Natrium, Cystein, Ascorbinsäure, α-Tocopherol oder auch Schwermetallionen komplexierende Verbindungen, wie EDTE-Dinatriumsalz;
Konservierungsmittel, wie antimikrobielle Mittel in bakteriostatischen oder fungistatischen Konzentrationen, beispielsweise Phenylmercurinitrat, Thiomersal, Benzethoniumchlorid, Benzalkoniumchlorid, Phenol, Kresol oder Chlorbutanol;
Geschmackskorrigentien, die insbesondere bei oralen Zubereitungen zur Anwendung kommen;
sowie weitere Träger- und Hilfsstoffe, die üblicherweise zur Formulierung der erfindungsgemäßen Arzneimittel verwendet werden können.

Die kosmetischen Mittel liegen insbesondere als Cremes, Gele, Salben oder Lösungen vor. Die kosmetischen Mittel werden in üblicher Weise formuliert, beispielsweise wie oben für topische pharmazeutische Mittel angegeben.

Die folgenden Beispiele beschreiben die Erfindung, ohne sie zu beschränken:

### Herstellung von Bis-(N-lactamyl)butanen (Beispiele 1 bis 4)

### Beispiel 1: (1,3-Bis-(1-pyrrolidonyl)buten)

In 25 g durch Kristallisation gereinigtes N-Vinylpyrrolidon wurde unter Stickstoff 10 sec. ein HCl-Gasstrom über eine Kapillare eingeleitet. Die Mischung wurde unter Stickstoffatmosphäre über Nacht bei 0° C aufbewahrt, wobei eine langsame Kristallisation einetzte. Zur Vervollständigung der Kristallisation wurden 10 ml Ether zugesetzt. 1,3-Bis-(1-pyrrolidonyl)buten kristallisierte in büschelförmigen Nadeln aus. Das Produkt wurde aus Ether-Aceton (1/5) umkristallisiert.
Schmelzpunkt: 72° C
Ausbeute: 78 %
- ¹³C-NMR (MHz, CDCl₃ ) : δ =: 174.01, 173.17, 125.14, 110.58, 46.08, 45.17, 42.31, 31.44, 31.15, 17.92, 17.39, 17.29 ppm.

### Beispiel 2: (1,3-Bis-(1-pyrrolidonyl)butan)

60 g 1,3-Bis-(1-pyrrolidonyl)buten, 1000 ml Eisessig und 10 g 10 %iges Palladium auf Kohle wurden unter Inertgas zusammengegeben. Anschließend wurde unter Rühren bei Raumtemperatur Wasserstoff eingeleitet. Die Wasserstoffaufnahme war nach etwa 30 min. beendet. Man ließ weitere 90 min. bei Raumtemperatur nachreagieren. Der Katalysator wurde abfiltriert und das Filtrat im Vakuum eingeengt. Das ölige Rohprodukt wurde anschließend im Vakuum destilliert.
Siedepunkt: 205-215° C (0,2 mbar)
Ausbeute: 90 %
- ¹³C-NMR (MHz, CDCl₃) : δ =: 174.58, 174.42, 47.16, 44.29, 41.87, 39.59, 31.45, 31.32, 30.91, 18.08, 17.94, 17.92 ppm.
MS (CI):
m/z = 224 (M⁺,70), 196 (60), 126 (100), 113 (95), 98 (50).
IR (Film): 0 [cm⁻¹] = 2995 (CH,s), 1680 (CO,s), 1425, 1286.

### Beispiel 3 (1,3-Bis-(1-caprolactamyl)buten)

Die Herstellung erfolgte ausgehend von 25 g durch Destillation gereinigtem N-Vinylcaprolactam gemäß der Vorschrift aus Beispiel 1.
Schmelzpunkt: 178° C
Ausbeute: 76 %
- ¹³C-NMR (MHz, CDCl₃) : δ =: 174.97, 173.98, 128.15, 110.02, 48.39, 45.14, 43.04, 37.56, 37.06, 29.93, 29.65, 29.25, 27.53, 23.46, 23.41, 17.13 ppm.

### Beispiel 4 (1,3-Bis-(1-caprolactamyl)butan)

Die Herstellung des 1,3-Bis-(1-caprolactamyl)butans erfolgte ausgehend von 60 g 1,3-Bis-(1-caprolactamyl)buten analog zu Beispiel 2.

Versuche zur Löslichkeit in 1,3-Bis(N-lactamyl)butanen (Beispiele 5 und 6)

### Beispiel 5 (1,3-Bis(N-lactamyl)butane als Solvens)

Zu 20 ml 1,3-Bis(pyrrolidon-1-yl)butan gab man den Wirkstoff in einer Menge, die über der in diesem Volumen lösbaren Menge dieses Wirkstoffes liegt. Es wurde 72 Stunden bei Raumtemperatur gerührt. Diese Zeit gewährleistete in aller Regel, daß die Sättigungslöslichkeit im Medium erreicht wurde. Anschließend wurde abfiltriert und im Filtrat UV-photometrisch der Gehalt an Wirkstoff bestimmt. Für folgende Wirkstoffe wurden die angegebenen Löslichkeiten ermittelt:

| | |
|---|---|
| Wirkstoff | maximale Löslichkeit bei 22° C |
| Prednison | 200 mg/ml |
| Sulfathiazol | 200 mg/ml |
| Trimethoprim | 150 mg/ml |
| Isopron | 190 mg/ml |
| Paracetamol | 250 mg/ml. |

### Beispiel 6 (1,3-Bis(N-lactamyl)butane als Cosolvens im Gemisch mit Wasser)

Zur Bestimmung der maximal löslichen Wirkstoffmenge wurde analog zu Beispiel 5 verfahren. Folgende Lösungen wurden getestet:
a) 1,3-Bis-(pyrrolidon-1-yl)butan (DHVP):Wasser (1:1)
b) 1,3-Bis-(pyrrolidon-1-yl)butan (DHVP):Wasser (1:3)
c) 1,3-Bis-(caprolactam-1-yl)butan(DHVC) : Wasser (1:1)
d) 1,3-Bis-(caprolactam-1-yl)butan (DHVC):Wasser (1:3)
e) 1,2-Propylenglykol (1,2-PG) :Wasser (1:1)
f) 1,2-Propylenglykol (1,2-PG) :Wasser (1:3)
g) Phophatpuffer pH 7,0.

Für den Wirkstoff Trimethoprim (E 1 % : 209,948 ml/g bei 288 nm in Methanol-Wasser 1:1) ergaben sich folgende Werte:

| Lösung (20 ml) | Einwaage [g] | pH-Wert | Extinktion | Verd. | gelöster Stoff [g] | Löslichkeit [ % ] |
|---|---|---|---|---|---|---|
| 50 % 1,2-PG | 0,3394 g | 8,05 | 0,62457 | 300 | 0,1785 | 0,89 |
| 25 % 1,2-PG | 0,3410 g | 7,54 | 0,58575 | 100 | 0,0558 | 0,28 |
| 50 % DHVP | 0,8113 g | 8,61 | 0,74501 | 1000 | 0,7097 | 3,55 |
| 25 % DHVP | 0,4128 g | 7,44 | 0,21658 | 1000 | 0,2063 | 1,03 |
| 50 % DHVC | 1,7779 g | 8,45 | 1,06760 | 1000 | 1,0170 | 5,09 |
| 25 % DHVC | 0,3892 g | 7,72 | 0,35504 | 1000 | 0,3382 | 1,69 |
| Phosphat-puffer pH 7,0 | 0,3712 g | 7,19 | 0,25705 | 100 | 0,0245 | 0,12 |

Für den Wirkstoff Theophyllin (E 1 % : 549,4755 ml/g bei 272 nm in Methanol-Wasser 1:1) ergaben sich folgende Werte:

| Lösung (20ml) | Einwaage [g] | pH-Wert | Extinktion | Verd. | gelöster Stoff [g] | Löslichkeit [%] |
|---|---|---|---|---|---|---|
| 50 % 1,2-PG | 0,9607 | 7,53 | 0,84715 | 1000 | 0,3083 | 1,54 |
| 25 % 1,2-PG | 0,9991 | 7,34 | 0,54880 | 1000 | 0,1998 | 1,00 |
| 50 % DHVP | 1,0473 | 7,75 | 1,01740 | 1000 | 0,3703 | 1,85 |
| 25 % DHVP | 0,9924 | 7,35 | 0,71689 | 1000 | 0,2609 | 1,30 |
| 50 % DHVC | 1,0307 | 7,53 | 0,50153 | 3000 | 0,5476 | 2,74 |
| 25 % DHVC | 1,0472 | 7,23 | 0,85304 | 1000 | 0,3105 | 1,55 |
| Phosphat-puffer pH 7,0 | 1,0014 | 7,09 | 0,32199 | 1000 | 0,1172 | 0,59 |

Für den Wirkstoff Paracetamol (E 1 % : 756,2982 ml/g bei 246 nm in Methanol-Wasser 1:1) ergaben sich folgende Werte:

| Lösung (20 ml) | Einwaage [g] | pH-Wert | Extinktion | Verd. | gelöster Stoff [g] | Löslichkeit [%] |
|---|---|---|---|---|---|---|
| 50 % 1,2-PG | 2,7846 | 7,72 | 0,48834 | 10000 | 1,2914 | 6,46 |
| 25 % 1,2-PG | 0,9787 | 7,42 | 0,69788 | 3000 | 0,5537 | 2,77 |
| 50 % DHVP | 8,2911 | 7,81 | 0,63400 | 30000 | 5,0298 | 25,15 |
| 25 % DHVP | 2,8011 | 7,42 | 0,80333 | 10000 | 2,1244 | 10,62 |
| Phosphat-puffer pH 7,0 | 1,0312 | 7,09 | 1,02790 | 1000 | 0,2718 | 1,36 |

Die ermittelten Löslichkeiten zeigen, daß 1,3-Bis-(pyrrolidon-1-yl)butan und 1,3-Bis-(caprolactam-1-yl)butan für hydrophile Wirkstoffe, wie Paracetamol, Theophyllin oder Trimethoprim, bessere Cosolventien im Gemisch mit Wasser darstellen als das vielfach verwendete Propylenglycol.

### Beispiel 7: Vergleich der Löslichkeit in 1,3-Bis-(pyrrolidon-1-yl)butan (DHVP) und 1,3-Bis-(pyrrolidon-1-yl)propan

Folgende Wirkstoffe wurden in 1,3-Bis-(pyrrolidon-1-yl)butan (DHVP) zu 30 Gew.-% gelöst:
Acetylcystein, Acetylsalicylsäure, Anipamil x HCl, Benzocain, Benzolidin, Captopril, Chloramphenicol, Clotrimazol, 5,5-Diphenylhydantoin, Fenofibrat, Flecainid, Furosemid, Gallopamil x HCl, Glibenclamid, Hydrochlorothiazid, Ibuprofen, Indometacin, Naphtidrofuryl, Nexopamil, Nifedipin, Nitrendipin, Nitrofurantoin, Paracetamol, (+)-Pseudoephidrin x HCl, Pentoxifyllin, Prazosin, Propafenon x HCl, Selegilin x HCl, Sulfamethazine, Sulfamethoxazol, Sulfathiazol, Sulindac und Tolbutamid.

Wurde anstatt des erfindungsgemäßen 1,3-Bis-(pyrrolidon-1-yl)butans das aus dem Stand der Technik bekannte 1,3-Bis-(pyrrolidon-1-yl)propan unter sonst gleichen Bedingungen verwendet, kam es bei den folgenden Wirkstoffen jedoch zur Rekristallisation, d.h. mit 1,3-Bis-(pyrrolidon-1-yl)propan konnten keine stabilen 30-% Lösungen dieser Wirkstoffe erhalten werden:

Benzocain, Chloramphenicol, Cyclosporin, Clotrimazol, 5,5-Diphenylhydantoin, Fenofibrat, Furosemid, Gallopamil x HCl, Glibenclamid, Hydrochlorothiazid, Indometacin, Naphtidrofuryl, Nifedipin, Nitrendipin, Paracetamol, Pentoxifyllin, Prazosin, Propafenon x HCl, Sulfamethazine, Sulfamethoxazol, Sulfathiazol, Sulindac und Tolbutamid.

### Pharmazeutische und kosmetische Formulierungen (Beispiele 8 bis 12)

### Beispiel 8

### Herstellung einer Oxytetracyclin-Injektionslösung:

| | |
|---|---|
| Oxytetracyclin | 22,65 g |
| Magnesiumoxid | 1,92 g |
| 1,3-Bis-(pyrrolidon-1-yl)butan | 40,00 g |
| Polyvinylpyrrolidon K 17 (z.B. Kollidon 17 PF) | 5,00 g |
| Magnesiumformaldehydsulfoxylat | 0,44 g |
| 2-Aminoethanol | 3,84 g |
| Wasser q.s. ad | 100,00 ml |

Wasser und 1,3-Bis-(pyrrolidon-1-yl)butan werden gemischt und Kollidon 17 PF darin gelöst; die Lösung wird auf 75°C erwärmt, Magnesiumformaldehydsulfoxylat zugegeben und unter Rühren gelöst. Nach Suspendieren des Magnesiumoxids wird Oxytetracyclin langsam eingerührt, bis eine klare Lösung resultiert. Nach Abkühlen wird der pH-Wert mit Aminoethanol auf 8,5 eingestellt.

### Beispiel 9

### Herstellung einer Trimethoprim-Sulfonamid-Kombination als Injektionslösung

| | |
|---|---|
| Trimethoprim | 2,0 g |
| Sulfadoxin | 10,0 g |
| 1,3-Bis-(pyrrolidon-l-yl)butan | 56,0 g |
| Wasser | 29,0 g |
| Natronlauge q.s. | (pH 8,5) |

Man löst die beiden Wirkstoffe in Bis-1,3-pyrrolidon-butan, gibt das Wasser zu und stellt den pH-Wert mit Natronlauge auf 8,5 ein.

### Beispiel 10

### Sulfamoxol-Trimethoprim-Injektionslösung

| | |
|---|---|
| Sulfamoxol | 4,0 g |
| Trimethoprim | 0,8 g |
| 1,3-Bis-(pyrrolidon-1-yl)butan | 30,0 g |
| Natriumsulfit | 0,4 g |
| Ethanol | 10,0 g |
| 1,2-Propylenglykol | 10,0 g |
| p-Hydroxybenzoesäureester | 0,2 g |
| Wasser für Injektionszwecke ad | 100,0 ml |

### Beispiel 11

### Gelzubereitung

| | |
|---|---|
| Ibuprofen | 10,0 g |
| 1,3-Bis-(pyrrolidon-1-yl)butan | 20,0 g |
| Lutrol F 127 (Polyethylenglykol) | 22,0 g |
| Lutrol F 68 (Polyethylenglykol) | 5,0 g |
| NaCl | 1,0 g |
| Wasser | 51,0 g |
| Eigenschaften: klares, farbloses | Gel |

### Beispiel 12

### Cremerezepturen vom Typ Öl-in-Wasser

### Hautpflegecreme

| | |
|---|---|
| Paraffinöl | 24,0 g |
| Cremophor S9 (PEG-9 Stearat) | 5,0 g |
| Bienenwachs | 6,0 g |
| Cutina®CP (Cetylpalmitat) | 2,0 g |
| 1,3-Bis-(pyrrolidon-1-yl)butan | 3,0 g |
| Wasser | 60,0 g |

### Feuchtigkeitscreme mit Collagen

| | |
|---|---|
| Cremophor S9 (PEG-9 Stearat) | 7,0 g |
| 1,3-Bis-(pyrrolidon-1-yl)butan | 1,5 g |
| Cetylalkohol | 6,0 g |
| Wollwachs | 3,0 g |
| Tegiloxan®350 (Dimethicone) Miglyol 812 (Capryl-Caprinsäure- | 2,0 g |
| Triglycerid) | 5,0 g |
| Karion® F (Sorbitol) | 3,0 g |
| Collagen | 5,0 g |
| (-)alpha-Bisabolol | 0,1 g |
| Konservierungsmittel | q.s. |
| Parfümöl | q.s. |
| Wasser | 67,4 g |

In diese Formulierungen können Wirkstoffe oder Vitamine eingebracht werden.

## Patentansprüche

1. Verwendung wenigstens eines 1,3-Bis-(N-lactamyl)propans der allgemeinen Formel I 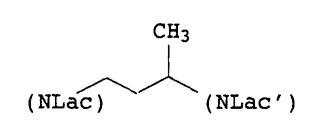 worin (NLac) und (NLac') gleich oder verschieden sind und für N-Lactamylreste stehen,
als Lösungsmittel in pharmazeutischen Mitteln.

2. Verwendung einer Verbindung nach Anspruch 1, wobei in der Formel I (NLac) und (NLac'), die gleich oder verschieden sein können, für N-Lactamylreste stehen, die ausgewählt sind unter Pyrrolidon, Piperidon, δ- oder ε-Caprolactam, Tetrahydro-1,4-oxazin-3-on oder Oxazolidin-4-on.

3. Verwendung einer Verbindung nach einem der vorhergehenden Ansprüche, wobei der Formel I (NLac) und (NLac') die gleiche Bedeutung besitzen.

4. Verwendung nach Anspruch 1 einer Verbindung, welche ausgewählt ist unter
1,3-Bis(pyrrolidon-1-yl)butan
1,3-Bis(piperidon-1-yl)butan
1,3-Bis(caprolactam-1-yl)butan
1,3-Bis(tetrahydro-1,4-oxazin-3-on-1-yl)butan
1,3-Bis(oxazolidin-4-on-3-yl)butan
1-(Caprolactam-1-yl)-3-(piperidon-1-yl)butan
3-(Caprolactam-1-yl)-1-(piperidon-1-yl)butan
1-(Caprolactam-1-yl)-3-(pyrrolidon-1-yl)butan
3-(Caprolactam-1-yl)-1-(pyrrolidon-1-yl)butan
1-(Caprolactam-1-yl)-3-(oxazolidin-4-on-3-yl)butan
3-(Caprolactam-1-yl)-1-(oxazolidin-4-on-3-yl)butan
1-(Caprolactam-1-yl)-3-(tetrahydro-1,4-oxazin-3-on-4-yl)butan
3-(Caprolactam-1-yl)-1-(tetrahydro-1,4-oxazin-3-on-4-yl)butan
1-(Oxazolidin-4-on-3-yl)-3-(piperidon-1-yl)butan
3-(Oxazolidin-4-on-3-yl)-1-(piperidon-1-yl)butan
1-(Oxazolidin-4-on-3-yl)-3-(pyrrolidon-1-yl)butan
3-(Oxazolidin-4-on-3-yl)-1-(pyrrolidon-1-yl)butan
1-(Oxazolidin-4-on-3-yl)-3-(tetrahydro-1,4-oxazin-3-on-4-yl)butan
3-(Oxazolidin-4-on-3-yl)-1-(tetrahydro-1,4-oxazin-3-on-4-yl)butan.
1-(Piperidon-1-yl)-3-(pyrrolidon-1-yl)butan
3-(Piperidon-1-yl)-1-(pyrrolidon-1-yl)butan
1-(Piperidon-1-yl)-3-(tetrahydro-1,4-oxazin-3-on-4-yl)butan
3-(Piperidon-1-yl)-1-(tetrahydro-1,4-oxazin-3-on-4-yl)butan
1-(Pyrrolidon-1-yl)-3-(tetrahydro-1,4-oxazin-3-on-4-yl)butan
3-(Pyrrolidon-1-yl)-1-(tetrahydro-1,4-oxazin-3-on-4-yl)butan.

5. Verwendung einer Verbindung nach einem der vorhergehenden Ansprüche der Formel I als Lösungsmittel in pharmazeutischen Mitteln zur systemischen und insbesondere parenteralen Anwendung.

6. Verwendung nach Anspruch 5 einer Verbindung der Formel I als Lösungsmittel in pharmazeutischen Mitteln zur Injektion oder im Füllgut von Kapseln.

7. Verwendung einer Verbindung nach einem der Ansprüche 1 bis 4 als Lösungsmittel in kosmetischen Mitteln.

8. 1,3-Bis(N-lactamyl)propan-Verbindungen der allgemeinen Formel Ia 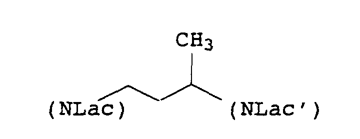 worin (NLac) und (NLac') gleich oder verschieden sind und für N-Lactamylreste stehen, ausgenommen 1,3-Bis(pyrrolidon-1-yl)butan.

9. Verbindungen nach Anspruch 8 der Formel I, worin (NLac) und (NLac'), die gleich oder verschieden sein können, für N-Lactamylreste stehen, die ausgewählt sind unter Pyrrolidon, Piperidon, δ- oder ε-Caprolactam, Tetrahydro-1,4-oxazin-3-on oder Oxazolidin-4-on.

10. Verbindung nach einem der Ansprüche 8 oder 9 der Formel I, worin (NLac) und (NLac') die gleiche Bedeutung besitzen.

11. Verbindung nach einem der Ansprüche 8 bis 10, nämlich
1,3-Bis(piperidon-1-yl)butan
1,3-Bis(caprolactam-1-yl)butan
1,3-Bis(tetrahydro-1,4-oxazin-3-on-1-yl)butan
1,3-Bis(oxazolidin-4-on-3-yl)butan
1-(Caprolactam-1-yl)-3-(piperidon-1-yl)butan
3-(Caprolactam-1-yl)-1-(piperidon-1-yl)butan
1-(Caprolactam-1-yl)-3-(pyrrolidon-1-yl)butan
3-(Caprolactam-1-yl)-1-(pyrrolidon-1-yl)butan
1-(Caprolactam-1-yl)-3-(oxazolidin-4-on-3-yl)butan
3-(Caprolactam-1-yl)-1-(oxazolidin-4-on-3-yl)butan
1-(Caprolactam-1-yl)-3-(tetrahydro-1,4-oxazin-3-on-4-yl)butan
3-(Caprolactam-1-yl)-1-(tetrahydro-1,4-oxazin-3-on-4-yl)butan
1-(Oxazolidin-4-on-3-yl)-3-(piperidon-1-yl)butan
3-(Oxazolidin-4-on-3-yl)-1-(piperidon-1-yl)butan
1-(Oxazolidin-4-on-3-yl)-3-(pyrrolidon-1-yl)butan
3-(Oxazolidin-4-on-3-yl)-1-(pyrrolidon-1-yl)butan
1-(Oxazolidin-4-on-3-yl)-3-(tetrahydro-1,4-oxazin-3-on-4-yl)butan
3-(Oxazolidin-4-on-3-yl)-1-(tetrahydro-1,4-oxazin-3-on-4-yl)butan
1-(Piperidon-1-yl)-3-(pyrrolidon-1-yl)butan
3-(Piperidon-1-yl)-1-(pyrrolidon-1-yl)butan
1-(Piperidon-1-yl)-3-(tetrahydro-1,4-oxazin-3-on-4-yl)butan
3-(Piperidon-1-yl)-1-(tetrahydro-1,4-oxazin-3-on-4-yl)butan
1-(Pyrrolidon-1-yl)-3-(tetrahydro-1,4-oxazin-3-on-4-yl)butan
3-(Pyrrolidon-1-yl)-1-(tetrahydro-1,4-oxazin-3-on-4-yl)butan.

12. Pharmazeutisches Mittel, enthaltend wenigstens eine Verbindung nach einem der Ansprüche 8 bis 11.

13. Kosmetisches Mittel, enthaltend wenigstens eine Verbindung nach einem der Ansprüche 8 bis 11.

## Claims

1. The use of at least one 1,3-bis(N-lactamyl)propane of the formula I 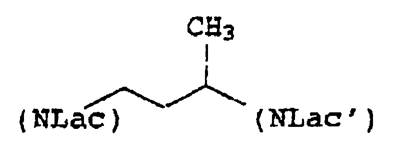 in which (NLac) and (NLac') are identical or different and are N-lactamyl radicals,
as solvent in pharmaceutical compositions.

2. The use of a compound as set forth in claim 1, where in formula I (NLac) and (NLac'), which may be identical or different, are N-lactamyl radicals which are selected from pyrrolidone, piperidone, δ- or ε-caprolactam, tetrahydro-1,4-oxazin-3-one or oxazolidin-4-one.

3. The use of a compound as set forth in either of the preceding claims, where in formula I (NLac) and (NLac') have the same meaning.

4. The use as claimed in claim 1 of a compound which is selected from
1,3-bis(pyrrolidon-1-yl)butane
1,3-bis(piperidon-1-yl)butane
1,3-bis(caprolactam-1-yl)butane
1,3-bis(tetrahydro-1,4-oxazin-3-on-1-yl)butane
1,3-bis(oxazolidin-4-on-3-yl)butane
1-(caprolactam-1-yl)-3-(piperidon-1-yl)butane
3-(caprolactam-1-yl)-1-(piperidon-1-yl)butane
1-(caprolactam-1-yl)-3-(pyrrolidon-1-yl)butane
3-(caprolactam-1-yl)-1-(pyrrolidon-1-yl)butane
1-(caprolactam-1-yl)-3-(oxazolidin-4-on-3-yl)-butane
3-(caprolactam-1-yl)-1-(oxazolidin-4-on-3-yl)-butane
1-(caprolactam-1-yl)-3-(tetrahydro-1,4-oxazin-3-on-4-yl)butane
3-(caprolactam-1-yl)-1-(tetrahydro-1,4-oxazin-3-on-4-yl)butane
1-(oxazolidin-4-on-3-yl)-3-(piperidon-1-yl)butane
3-(oxazolidin-4-on-3-yl)-1-(piperidon-1-yl)butane
1-(oxazolidin-4-on-3-yl)-3-(pyrrolidon-1-yl)butane
3-(oxazolidin-4-on-3-yl)-1-(pyrrolidon-1-yl)butane
1-(oxazolidin-4-on-3-yl)-3-(tetrahydro-1,4-oxazin-3-on-4-yl)butane
3-(oxazolidin-4-on-3-yl)-1-(tetrahydro-1,4-oxazin-3-on-4-yl)butane
1-(piperidon-1-yl)-3-(pyrrolidon-1-yl)butane
3-(piperidon-1-yl)-1-(pyrrolidon-1-yl)butane
1-(piperidon-1-yl)-3-(tetrahydro-1,4-oxazin-3-on-4-yl)butane
3-(piperidon-1-yl)-1-(tetrahydro-1,4-oxazin-3-on-4-yl)butane
1-(pyrrolidon-l-yl)-3-(tetrahydro-1,4-oxazin-3-on-4-yl)butane
3-(pyrrolidon-1-yl)-1-(tetrahydro-1,4-oxazin-3-on-4-yl)butane.

5. The use of a compound as set forth in any of the preceding claims of the formula I as solvent in pharmaceutical compositions for systemic and, in particular, parenteral administration.

6. The use as claimed in claim 5 of a compound of the formula I as solvent in pharmaceutical compositions for injection or in the contents of capsules.

7. The use of a compound as set forth in any of claims 1 to 4 as solvent in cosmetic compositions.

8. A 1,3-bis(N-lactamyl)propane compound of the formula Ia 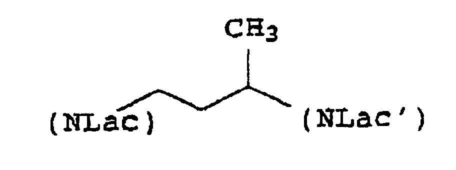 in which (NLac) and (NLac') are identical or different and are N-lactamyl radicals, excepting 1,3-bis(pyrrolidon-1-yl)butane.

9. A compound as claimed in claim 8 of the formula I, in which (NLac) and (NLac'), which may be identical or different, are N-lactamyl radicals which are selected from pyrrolidone, piperidone, δ- or ε-caprolactam, tetrahydro-1,4-oxazin-3-one or oxazolidin-4-one.

10. A compound as claimed in either of claims 8 and 9 of the formula I, in which (NLac) and (NLac') have the same meaning.

11. A compound as claimed in any of claims 8 to 10, namely
1,3-bis(piperidon-1-yl)butane
1,3-bis(caprolactam-1-yl)butane
1,3-bis(tetrahydro-1,4-oxazin-3-on-1-yl)butane
1,3-bis(oxazolidin-4-on-3-yl)butane
1-(caprolactam-1-yl)-3-(piperidon-1-yl)butane
3-(caprolactam-1-yl)-1-(piperidon-1-yl)butane
1-(caprolactam-1-yl)-3-(pyrrolidon-1-yl)butane
3-(caprolactam-1-yl)-1-(pyrrolidon-1-yl)butane
1-(caprolactam-1-yl)-3-(oxazolidin-4-on-3-yl)-butane
3-(caprolactam-1-yl)-1-(oxazolidin-4-on-3-yl)-butane
1-(caprolactam-1-yl)-3-(tetrahydro-1,4-oxazin-3-on-4-yl)butane
3-(caprolactam-1-yl)-1-(tetrahydro-1,4-oxazin-3-on-4-yl)butane
1-(oxazolidin-4-on-3-yl)-3-(piperidon-1-yl)butane
3-(oxazolidin-4-on-3-yl)-1-(piperidon-1-yl)butane
1-(oxazolidin-4-on-3-yl)-3-(pyrrolidon-1-yl)butane
3-(oxazolidin-4-on-3-yl)-1-(pyrrolidon-1-yl)butane
1-(oxazolidin-4-on-3-yl)-3-(tetrahydro-1,4-oxazin-3-on-4-yl)butane
3-(oxazolidin-4-on-3-yl)-1-(tetrahydro-1,4-oxazin-3-on-4-yl)butane
1-(piperidon-1-yl)-3-(pyrrolidon-1-yl)butane
3-(piperidon-1-yl)-1-(pyrrolidon-1-yl)butane
1-(piperidon-1-yl)-3-(tetrahydro-1,4-oxazin-3-on-4-yl)butane
3-(piperidon-1-yl)-1-(tetrahydro-1,4-oxazin-3-on-4-yl)butane
1-(pyrrolidon-1-yl)-3-(tetrahydro-1,4-oxazin-3-on-4-yl)butane
3-(pyrrolidon-1-yl)-1-(tetrahydro-1,4-oxazin-3-on-4-yl)butane.

12. A pharmaceutical composition comprising at least one compound as claimed in any of claims 8 to 11.

13. A cosmetic composition comprising at least one compound as claimed in any of claims 8 to 11.

## Revendications

1. Utilisation d'au moins un 1,3-bis-(N-lactamyl)propane de formule générale I 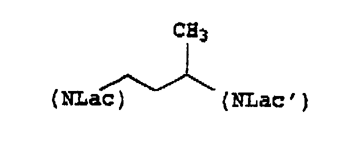 où (NLac) et (NLac') sont identiques ou différents et désignent des restes N-lactamyle,
comme solvant dans des produits pharmaceutiques.

2. Utilisation d'un composé selon la revendication 1, dans laquelle dans la formule I (NLac) et (NLac'), qui peuvent être identiques ou différents, désignent des restes N-lactamyle qui sont choisis parmi la pyrrolidone, la pipéridone, le δ- ou l'ε-caprolactame, la tétrahydro-1,4-oxazine-3-one ou l'oxazolidine-4-one,

3. Utilisation d'un composé selon l'une des revendications précédentes, dans laquelle dans la formule I (NLac) et (NLac') ont la même signification.

4. Utilisation selon la revendication 1 d'un composé qui est choisi parmi les suivants
1,3-bis(pyrrolidone-1-yl)butane
1,3-bis(pipéridone-1-yl)butane
1,3-bis(caprolactame-1-yl)butane
1,3-bis(tétrahydro-1,4-oxazine-3-one-1-yl)butane
1,3-bis(oxazolidine-4-one-1-yl)butane
1-(caprolactame-1-yl)-3-(pipéridone-1-yl)butane
3-(caprolactame-1-yl)-1-(pipéridone-1-yl)butane
1-(caprolactame-1-yl)-3-(pyrrolidone-1-yl)butane
3-(caprolactame-1-yl)-1-(pyrrolidone-1-yl)butane
1-(caprolactame-1-yl)-3-(oxazolidine-4-one-3-yl)-butane
3-(caprolactame-1-yl)-1-(oxazolidine-4-one-3-yl)-butane
1-(caprolactame-1-yl)-3-(tétrahydro-1,4-oxazine-3-one-4-yl)butane
3-(caprolactame-1-yl)-1-(tétrahydro-1,4-oxazine-3-one-4-yl)butane
1-(oxazolidine-4-one-3-yl)-3-(pipéridone-1-yl)butane
3-(oxazolidine-4-one-3-yl)-1-(pipéridone-1-yl)butane
1-(oxazolidine-4-one-3-yl)-3-(pyrrolidone-1-yl)butane
3-(oxazolidine-4-one-3-yl)-1-(pyrrolidone-1-yl)butane
1-(oxazolidine-4-one-3-yl)-3-(tétrahydro-1,4-oxazine-3-one-4-yl)butane
3-(oxazolidine-4-one-3-yl)-1-(tétrahydro-1,4-oxazine-3-one-4-yl)butane
1-(pipéridone-1-yl)-3-(pyrrolidone-1-yl)butane
3-(pipéridone-1-yl)-1-(pyrrolidone-1-yl)butane
1-(pipéridone-1-yl)-3-(tétrahydro-1,4-oxazine-3-one-4-yl)butane
3-(pipéridone-1-yl)-1-(tétrahydro-1,4-oxazine-3-one-4-yl)butane
1-(pyrrolidone-1-yl)-3-(tétrahydro-1,4-oxazine-3-one-4-yl)butane
3-(pyrrolidone-1-yl)-1-(tétrahydro-1,4-oxazine-3-one-4-yl)butane.

5. Utilisation d'un composé selon l'une des revendications précédentes de formule I comme solvant dans des produits pharmaceutiques pour application systémique et en particulier parentérale.

6. Utilisation selon la revendication 5 d'un composé de formule I comme solvant dans des produits pharmaceutiques pour injection ou dans la matière de remplissage de capsules.

7. Utilisation d'un composé selon l'une des revendications 1 à 4 comme solvant dans des produits cosmétiques.

8. Composés de 1,3-bis(N-lactamyl)propane de formule générale Ia 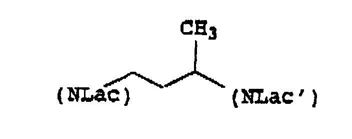 où (NLac) et (NLac') sont identiques ou différents et désignent des restes N-lactamyle, à l'exception du 1,3-bis(pyrrolidone-1-yl)butane.

9. Composés selon la revendication 8 de formule I, dans lesquels (NLac) et (NLac'), qui peuvent être identiques ou différents, désignent des restes N-lactamyle qui sont choisis parmi la pyrrolidone, la pipéridone, le δ- ou l'ε-caprolactame, la tétrahydro-1,4-oxazine-3-one ou l'oxazolidine-4-one.

10. Composé selon l'une des revendications 8 ou 9 de formule I, dans lequel (NLac) et (NLac') ont la même signification.

11. Composé selon l'une des revendications 8 à 10, à savoir
1,3-bis(pipéridone-1-yl)butane
1,3-bis(caprolactame-1-yl)butane
1,3-bis(tétrahydro-1,4-oxazine-3-one-l-yl)butane
1,3-bis(oxazolidine-4-one-1-yl)butane
1-(caprolactame-1-yl)-3-(pipéridone-1-yl)butane
3-(caprolactame-1-yl)-1-(pipéridone-1-yl)butane
1-(caprolactame-1-yl)-3-(pyrrolidone-1-yl)butane
3-(caprolactame-1-yl)-1-(pyrrolidone-1-yl)butane
1-(caprolactame-1-yl)-3-(oxazolidine-4-one-3-yl)-butane
3-(caprolactame-1-yl)-1-(oxazolidine-4-one-3-yl)-butane
1-(caprolactame-1-yl)-3-(tétrahydro-1,4-oxazine-3-one-4-yl)butane
3-(caprolactame-1-yl)-1-(tétrahydro-1,4-oxazine-3-one-4-yl)butane
1-(oxazolidine-4-one-3-yl)-3-(pipéridone-1-yl)butane
3-(oxazolidine-4-one-3-yl)-1-(pipéridone-1-yl)butane
1-(oxazolidine-4-one-3-yl)-3-(pyrrolidone-1-yl)butane
3-(oxazolidine-4-one-3-yl)-1-(pyrrolidone-1-yl)butane
1-(oxazolidine-4-one-3-yl)-3-(tétrahydro-1,4-oxazine-3-one-4-yl)butane
3-(oxazolidine-4-one-3-yl)-1-(tétrahydro-1,4-oxazine-3-one-4-yl)butane
1-(pipéridone-1-yl)-3-(pyrrolidone-1-yl)butane
3-(pipéridone-1-yl)-1-(pyrrolidone-1-yl)butane
1-(pipéridone-1-yl)-3-(tétrahydro-1,4-oxazine-3-one-4-yl)butane
3-(pipéridone-1-yl)-1-(tétrahydro-1,4-oxazine-3-one-4-yl)butane
1-(pyrrolidone-1-yl)-3-(tétrahydro-1,4-oxazine-3-one-4-yl)butane
3-(pyrrolidone-1-yl)-1-(tétrahydro-1,4-oxazine-3-one-4-yl)butane.

12. Produit pharmaceutique, contenant au moins un composé selon l'une des revendications 8 à 11.

13. Produit cosmétique, contenant au moins un composé selon l'une des revendications 8 à 11.
